# EUROPÄISCHE PATENTSCHRIFT

(11) **EP 0 508 191 B1**
(45) Veröffentlichungstag und Bekanntmachung des Hinweises auf die Patenterteilung: **29.05.1996**
(21) Anmeldenummer: 92104938.3
(22) Anmeldetag: 21.03.1992
(51) Int. Cl.: C07D 231/12, A01N 43/56, C07D 231/16

(54) **Verfahren zur Herstellung von 1-Carbamoyl-pyrazolen**
Process for the production of 1-carbamoyl-pyrazoles
Procédé de préparation de carbamoyl-1-pyrazoles

(30) Priorität: 12.04.1991 DE 4111922
(43) Veröffentlichungstag der Anmeldung: 14.10.1992
(73) Patentinhaber: BASF Aktiengesellschaft, D-67063 Ludwigshafen (DE)
(72) Erfinder: Kaestner, Ralf, Dr., W-6700 Ludwigshafen (DE); Rieber, Norbert, Dr., W-6800 Mannheim 51 (DE); Merger, Franz, Dr., W-6710 Frankenthal (DE)

(56) Entgegenhaltungen:
- DD-A- 210 541
- DD-A- 249 409
- DD-A- 259 860
- DE-A- 2 745 833
- DE-A- 3 815 788
- US-A- 3 625 948

## Beschreibung

Die vorliegende Erfindung betrifft ein Verfahren zur Herstellung von 1-Carbamoyl-pyrazol I 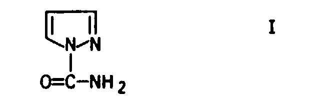 und dessen Derivaten durch Umsetzung von Pyrazol II 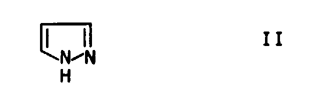 bzw. dessen Derivaten.

Aus der Literatur sind verschiedene Verfahren zur Herstellung von 1-Carbamoyl-pyrazolen bekannt. Beispielsweise wird die Umsetzung von Pyrazolderivaten mit Alkalimetallcyanaten in schwach alkalischer Lösung (DE-A 38 15 788, DD-A 259,860, DD-A 249,409 und DD-A 210,541) und die zweistufige Umsetzung von Pyrazolen zunächst mit Phosgen und anschließend mit Ammoniak (Auwers et al., J. prakt. Chem. 110, 253 (1925)) beschrieben.

Diese bekannten Verfahren sind im Hinblick auf eine großtechnische Anwendung wegen der hochgiftigen Ausgangsstoffe (Alkalimetallcyanate, Phosgen und Ammoniak) und wegen der Entsorgung der bei derartigen Umsetzungen gebildeten Salze problematisch.

Des weiteren ist bekannt, daß die Umsetzung aliphatischer Amine mit Harnstoff zu entsprechenden Carbamoylderivaten führt (US-A 3,625,948, US-A 4,766,115, US-A 4,833,245). Der Versuch, die hierfür beschriebenen Bedingungen auf die Herstellung von 1-Carbamoyl-pyrazolen zu übertragen, führt jedoch, wegen der vergleichsweise geringeren Nucleophilie der Pyrazole auf der einen Seite und der geringen Reaktivität des Harnstoffs auf der anderen Seite, nicht zum gewünschten Erfolg.

Der Erfindung lag die Aufgabe zugrunde, 1-Carbamoyl-pyrazol und dessen Derivate auf technisch einfache und wirtschaftliche Weise zugänglich zu machen.

Entsprechend dieser Aufgabe wurde ein Verfahren zur Herstellung von 1-Carbamoyl-pyrazol I 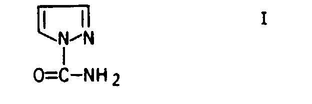 und dessen Derivaten durch Umsetzung von Pyrazol II 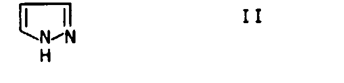 bzw. dessen Derivaten gefunden, welches dadurch gekennzeichnet ist, daß man II bzw. dessen Derivate bei Temperaturen oberhalb von 30°C mit Harnstoff umsetzt.

Die Reaktion setzt im allgemeinen bei Temperaturen oberhalb von 30°C ein. Sie läuft bei Temperaturen von 50°C mit ausreichender Geschwindigkeit ab. Im allgemeinen sollten jedoch 250°C nicht überschritten werden, da die zu der gewünschten Umsetzung in Konkurrenz stehende Reaktion von Harnstoff zu Biuret mit höheren Temperaturen stärker ins Gewicht fällt.

Aus diesen Erwägungen sollte die Umsetzungstemperatur im allgemeinen bei 40 bis 200°C, vorzugsweise bei 50 bis 180°C, insbesondere bei 70 bis 150°C liegen.

Da der bei der Umsetzung von Pyrazolen mit Harnstoff frei werdende Ammoniak infolge seiner höheren Basizität verglichen mit Pyrazol in der Lage ist, das bereits gebildete 1-Carbamoyl-pyrazol zu Spalten und somit die Ausbeute herabzusetzen, empfiehlt es sich, ihn aus dem Reaktionsgemisch zu entfernen.

Dies geschieht im allgemeinen kontinuierlich in verfahrenstechnisch üblicher Weise dadurch, daß man ein inertes Gas durch die Reaktionsmischung leitet oder dadurch, daß man die Umsetzung bei vermindertem Druck durchführt.

Das Verhältnis, in dem die Vorprodukte Pyrazol und Harnstoff miteinander umgesetzt werden, kann in weiten Bereichen (0,01 bis 1 mol-Äq. Harnstoff bezogen auf das Pyrazol) variieren.

Im allgemeinen wird Harnstoff im Unterschuß bezogen auf das Pyrazol verwendet, da bei den Umsetzungstemperaturen überschüssiger Harnstoff zu Biuret reagieren würde.

Üblicherweise verwendet man den Harnstoff in Mengen von 0,01 bis 0,95 mol-Äq., vorzugsweise 0,03 bis 0,8 mol-Äq., insbesondere 0,05 bis 0,5 mol-Äq. bezogen auf das eingesetzte Pyrazol.

Eine weitere Möglichkeit, die Bildung von Biuret während der Umsetzung zu unterdrücken, beruht darauf, den Harnstoff während der Umsetzung bei erhöhter Temperatur dosiert zuzugeben, da auf diese Art und Weise in der Reaktionsmischung kein Überschuß an Harnstoff entsteht. Die dosierte Zugabe kann kontinuierlich oder diskontinuierlich, in Substanz oder in Lösung erfolgen.

Die Umsetzung von Pyrazolen mit Harnstoff wird üblicherweise ohne Zusatz eines Lösungs- oder Verdünnungsmittels in der Schmelze durchgeführt.

Nach den bisherigen Untersuchungen kann die Verwendung einer Lewis-Säure wie insbesondere Eisen-III-salze zu einer Steigerung der Ausbeute führen.

Man erhält das 1-Carbamoyl-pyrazol nach dem Erkalten der Reaktionsmischung in üblicher Weise:
- Sofern die Umsetzung in der Schmelze durchgeführt wurde beispielsweise dadurch, daß man die Mischung mit Wasser aufnimmt und den unlöslichen Rückstand, der das Produkt enthält, umkristallisiert, destilliert, extrahiert oder chromatographiert.

Es kann hierbei empfehlenswert sein, das unumgesetzte Pyrazol vor der Aufarbeitung der Reaktionsmischung destillativ zu entfernen.
- Sofern man die Umsetzung in einem Lösungsmittel durchführt, empfiehlt es sich, zunächst das Lösungsmittel und das nicht umgesetzte Pyrazol destillativ zu entfernen, bevor man die Reaktionsmischung mit Wasser versetzt.

Das erfindungsgemäße Verfahren eignet sich zur Herstellung von 1-Carbamoyl-pyrazol und substituierten 1-Carbamoyl-pyrazolen aus den entsprechenden Pyrazolen, insbesondere solchen der allgemeinen Formel Ia 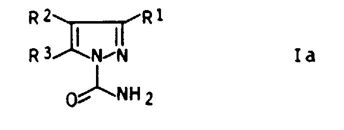 in der R¹, R und R³ Wasserstoff, Nitro, Halogen oder C-organische Reste, sowie über Heteroatome wie Sauerstoff, Schwefel und Stickstoff gebundene C-organische Reste und Carboxylgruppen bedeuten, wobei die Natur der Substituenten nach den bisherigen Erkenntnissen nur von geringem Einfluß ist.

Als Reste R¹, R und R³ kommen neben Wasserstoff und Nitrogruppen vorzugsweise ein bis drei der folgenden Gruppen in Betracht:
Halogenatome wie insbesondere Fluor, Chlor, Brom und Jod;
Alkylgruppen wie insbesondere Methyl, Ethyl, Propyl, 1-Methylethyl, Butyl, 1-Methylpropyl, 2-Methylpropyl und 1,1-Dimethylethyl;
Cycloalkylgruppen wie insbesondere Cyclopropyl, Cyclobutyl, Cyclopentyl und Cyclohexyl;
Alkenylgruppen wie insbesondere Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl und 1-Ethyl-ethenyl;
Alkinylgruppen wie insbesondere Ethinyl, 1-Propinyl, 2-Propinyl, 1-Butinyl, 2-Butinyl, 3-Butinyl und 1-Methyl-2-propinyl;
Arylgruppen oder Heteroarylgruppen wie insbesondere Phenyl;

Die genannten C-organischen Reste können ihrerseits über Heteroatome wie Sauerstoff, Schwefel oder Stickstoff an den Pyrazolring gebunden sein, durch Heteroatome wie Sauerstoff, Schwefel und Stickstoff unterbrochen sein oder weitere inerte Substituenten wie Halogen, Nitro, Aryl, Sulfonyl, Alkylsulfonyl, Arylsulfonyl und Carboxyl tragen, soweit sie sich unter den Reaktionsbedingungen inert verhalten.

Die als Ausgangsverbindungen dienenden Pyrazole IIa sind bekannt oder nach bekannten Methoden erhältlich (DE-A 39 18 979).

Das erfindungsgemäße Verfahren ermöglicht außerdem die Herstellung von 1-Carbamoyl-pyrazolen der allgemeinen Formel Ib, 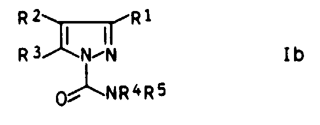 in der R¹, R und R³ die vorstehend gegebene Bedeutung haben und R⁴ und R⁵ für die o.g. C-organischen Reste stehen.

In diesem Fall setzt man unter den vorstehend beschriebenen Bedingungen eine Verbindung der allgemeinen Formel IIa mit einem symmetrisch substituierten Harnstoff der allgemeinen Formel IIIa um.

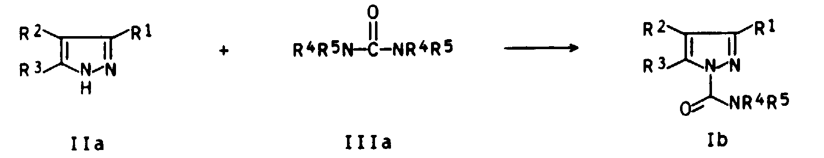

Die nach dem erfindungsgemäßen Verfahren leichter zugänglichen 1-Carbamoyl-pyrazole sind als Bioregulatoren bekannt (z.B. DE-A 27 45 833). Insbesondere 1-Carbamoyl-3-methyl-pyrazol (CMP) ist ein wertvolles Produkt, welches zur Hemmung der Nitrifikation von Ammonium-Stickstoff in Kulturböden in der Landwirtschaft verwendet wird.

### Verfahrensbeispiele

### Beispiel 1

Eine Mischung von 40 g (0,488 mol) 3-Methyl-pyrazol und 3,0 g (0,048 mol) Harnstoff wurde auf 110°C erhitzt. Bei dieser Temperatur wurde 30 Stunden lang ein Stickstoffstrom von 10 l/h durch die Reaktionsmischung geleitet. Sie wurde 5 Stunden auf -10°C abgekühlt. Dabei fielen 4,1 g (0,033 mol) 1-Carbamoyl-3-methyl-pyrazol vom Schmelzpunkt 127°C aus (Ausbeute: 68 % d. Th. bez. auf Harnstoff).

### Beispiel 2

Eine Mischung von 20 g (0,244 mol) 3-Methyl-pyrazol und 1,5 g (0,024 mol) Harnstoff wurde auf 100°C erhitzt. Nach 24 Stunden wurden weitere 1,5 g Harnstoff zugegeben und weitere 24 Stunden bei 100°C gehalten. Während der gesamten Reaktionszeit wurde ein Stickstoffstrom von 1 l/h durch die Reaktionsmischung geleitet. Nach Abkühlen wurden 81,2 Gew.-% 3-Methylpyrazol, 6,0 Gew.-% Harnstoff, 13,8 Gew.-% 1-Carbamoyl-3-methyl-pyrazol und 0,62 Gew.-% Biuret gefunden (HPLC-Analyse). Der Harnstoff-Umsatz betrug 56 %, die Ausbeute von 1-Carbamoyl-3-methyl-pyrazol bezogen auf Harnstoff 48 %, die Selektivität zu 1-Carbamoyl-3-methyl-pyrazol bezogen auf Harnstoff 86 % und die Selektivität zu Biuret bezogen auf Harnstoff 5 %.

### Beispiel 3

Eine Mischung von 20 g (0,24 mol) 3-Methyl-pyrazol und 14,7 g (0,24 mol) Harnstoff wurde auf 100°C erhitzt und bei dieser Temperatur 32 Stunden lang ein Stickstoffstrom von 5 l/h hindurchgeleitet. Beim Lösen in 100 ml Wasser blieben 1,7 g eines Feststoffs zurueck. Nach Umkristallisation aus Chloroform erhielt man 1,3 g 1-Carbamoyl-3-methyl-pyrazol vom Schmelzpunkt 125°C.

### Beispiel 4

Eine Mischung von 20 g (0,29 mol) Pyrazol und 1,8 g (0,03 mol) Harnstoff wurde geschmolzen und bei 100°C 10 Stunden lang ein Stickstoffstrom von 10 l/h hindurchgeleitet. Nach ¹H-NMR lagen in der abgekuehlten Schmelze 7,80 Gew.-% Pyrazol-1-carbonamid vor (8,3 ppm, 1H; 7,9 ppm, 2H; 7,8 ppm, 1H und 6.5 ppm, 1H). Das entspricht einer Ausbeute von 47 % d. Th. bez. auf Harnstoff.

### Beispiel 5

51,3 g (0,5 mol) 4-Chlor-pyrazol wurden geschmolzen und die Schmelze bei 120°C gehalten. 6,06 g (0,1 mol) Harnstoff wurden darin gelöst. Durch die Reaktionsmischung wurde bei der angegebenen Temperatur 22 Stunden lang ein Stickstoffstrom von 20 l/h geleitet. Die Schmelze wurde abgekühlt und in 175 ml Methyl-tert.-butylether gelöst. Der unlösliche Rückstand wurde abfiltriert und mit 70 ml Wasser gewaschen. Es blieben 6,2 g 1-Carbamoyl-4-chlor-pyrazol mit einem Schmelzpunkt von 191°C zurück (Ausbeute: 43 % d. Th. bez. auf Harnstoff).

## Patentansprüche

1. Verfahren zur Herstellung von 1-Carbamoyl-pyrazol I 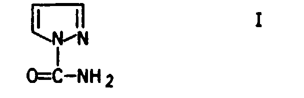 und dessen Derivaten durch Umsetzung von Pyrazol II 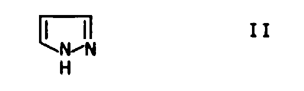 bzw. dessen Derivaten, dadurch gekennzeichnet, daß man II bzw. dessen Derivate bei Temperaturen oberhalb von 30°C mit Harnstoff umsetzt.

2. Verfahren zur Herstellung von 1-Carbamoyl-pyrazol I und dessen Derivaten nach Anspruch 1, dadurch gekennzeichnet, daß man die Umsetzung bei Temperaturen von 30 bis 250°C durchführt.

3. Verfahren zur Herstellung von 1-Carbamoyl-pyrazol I und dessen Derivaten nach Anspruch 1, dadurch gekennzeichnet, daß man den bei der Reaktion entstehenden Ammoniak kontinuierlich aus dem Reaktionsgemisch entfernt.

4. Verfahren zur Herstellung von 1-Carbamoyl-pyrazol I und dessen Derivaten nach Anspruch 1, dadurch gekennzeichnet, daß man den bei der Reaktion entstehenden Ammoniak während der Umsetzung aus dem Reaktionsgemisch entfernt.

5. Verfahren zur Herstellung von 1-Carbamoyl-pyrazol I und dessen Derivaten nach Anspruch 1, dadurch gekennzeichnet, daß man die Umsetzung bei vermindertem Druck durchführt.

6. Verfahren zur Herstellung von 1-Carbamoyl-pyrazol I und dessen Derivaten nach Anspruch 1, dadurch gekennzeichnet, daß man 0,01 bis 1 mol-Äq. Harnstoff bezogen auf das Pyrazol einsetzt.

7. Verfahren zur Herstellung von 1-Carbamoyl-3-methyl-pyrazol I nach Anspruch 1, dadurch gekennzeichnet, daß man 3-Methyl-pyrazol mit Harnstoff umsetzt.

8. Verfahren zur Herstellung von 1-Carbamoyl-3-methyl-pyrazol nach Anspruch 7, dadurch gekennzeichnet, daß man den bei der Reaktion entstehenden Ammoniak kontinuierlich aus dem Reaktionsgemisch entfernt.

## Claims

1. A process for the preparation of 1-carbamoylpyrazole I 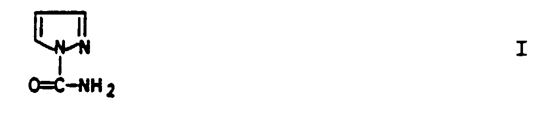 or a derivative thereof by reacting pyrazole II 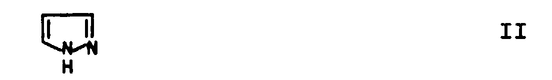 or a derivative thereof, wherein II or the derivative thereof is reacted with urea at above 30°C.

2. A process for the preparation of 1-carbamoylpyrazole I or a derivative thereof as claimed in claim 1, wherein the reaction is carried out at from 30 to 250°C.

3. A process for the preparation of 1-carbamoylpyrazole I or a derivative thereof as claimed in claim 1, wherein the ammonia formed in the reaction is removed continuously from the reaction mixture.

4. A process for the preparation of 1-carbamoylpyrazole I or a derivative thereof as claimed in claim 1, wherein the ammonia formed in the reaction is removed from the reaction mixture during the reaction.

5. A process for the preparation of 1-carbamoylpyrazole I or a derivative thereof as claimed in claim 1, wherein the reaction is carried out under reduced pressure.

6. A process for the preparation of 1-carbamoylpyrazole I or a derivative thereof as claimed in claim 1, wherein from 0.01 to 1 mole equivalent of urea, based on the pyrazole, is used.

7. A process for the preparation of 1-carbamoyl-3-methylpyrazole I as claimed in claim 1, wherein 3-methylpyrazole is reacted with urea.

8. A process for the preparation of 1-carbamoyl-3-methylpyrazole as claimed in claim 7, wherein the ammonia formed in the reaction is removed continuously from the reaction mixture.

## Revendications

1. Procédé pour la préparation du 1-carbamoylpyrazole I 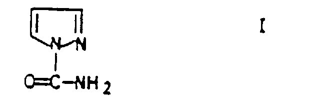 et ses dérivés, par conversion du pyrazole II 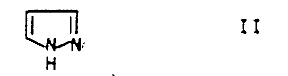 et respectivement de ses dérivés, caractérisé en ce que l'on fait réagir Il ou ses dérivés avec l'urée à des températures supérieures à 30°C.

2. Procédé de préparation du 1-carbamoylpyrazole I et de ses dérivés selon la revendication 1, caractérisé en ce que la réaction est effectuée à des températures de 30 à 250°C.

3. Procédé de préparation du 1-carbamoylpyrazole I et de ses dérivés selon la revendication 1, caractérisé en ce que l'on élimine en continu du mélange de réaction l'ammoniac formé dans la réaction.

4. Procédé de préparation du 1-carbamoylpyrazole I et de ses dérivés selon la revendication 1, caractérisé en ce que l'on élimine l'ammoniac formé du mélange de réaction en cours de réaction.

5. Procédé de préparation du 1-carbamoylpyrazole I et de ses dérivés selon la revendication 1, caractérisé en ce que la réaction est effectuée sous pression réduite.

6. Procédé de préparation du 1-carbamoylpyrazole I et de ses dérivés selon la revendication 1, caractérisé en ce que l'on met en oeuvre de 0,01 à 1 équivalent molaire d'urée par rapport au pyrazole.

7. Procédé de préparation du 1-carbamoyl-3-méthylpyrazole I selon la revendication 1, caractérisé en ce que l'on fait réagir le 3-méthylpyrazole avec l'urée.

8. Procédé de préparation du 1-carbamoyl-3-méthylpyrazole selon la revendication 7, caractérisé en ce que l'on élimine en continu du mélange de réaction l'ammoniac formé dans la réaction.
